# EUROPEAN PATENT SPECIFICATION

(11) **EP 4 403 559 B1**
(45) Date of publication and mention of the grant of the patent: **28.01.2026**
(21) Application number: 22869074.9
(22) Date of filing: 06.09.2022
(51) Int. Cl.: C07D 493/20, C07D 493/22, C07D 493/10, A61K 31/365, A61P 35/00

(54) **GUAIANE SESQUITERPENE POLYMER AND PREPARATION METHOD THEREFOR AND USE THEREOF**
GUAIAN-SESQUITERPEN-POLYMER UND HERSTELLUNGSVERFAHREN DAFÜR UND VERWENDUNG DAVON
POLYMÈRE DE SESQUITERPÈNE DE GUAÏANE ET PROCÉDÉ DE PRÉPARATION S'Y RAPPORTANT ET UTILISATION ASSOCIÉE

(30) Priority: 15.09.2021 CN 202111095790
(43) Date of publication of application: 24.07.2024
(73) Proprietor: Kunming Institute of Botany, Chinese Academy of Sciences, Panlong District Kunming Yunnan 650201 (CN)
(72) Inventor: CHEN, Jijun, Kunming, Yunnan 650201 (CN); LI, Tianze, Kunming, Yunnan 650201 (CN); MA, Yunbao, Kunming, Yunnan 650201 (CN); GENG, Changan, Kunming, Yunnan 650201 (CN); ZHANG, Xuemei, Kunming, Yunnan 650201 (CN)
(74) Representative: MacLachlan IP Limited
(86) International application number: PCT/CN2022/117191
(87) International publication number: WO 2023/040703

(56) References cited:
- CN-A- 101 585 841
- CN-A- 104 311 572
- CN-A- 112 225 746
- CN-A- 112 225 746
- CN-A- 113 788 840
- BOHLMANN FERDINAND ET AL: "Dimeric guaianolides from Artemisia sieversiana", vol. 24, no. 5, 1 January 1985 (1985-01-01), pages 1009 - 1015, XP093243268, ISSN: 0031-9422, Retrieved from the Internet <URL:https://doi.org/10.1016/S0031-9422(00)83172-6> DOI: 10.1016/S0031-9422(00)83172-6
- GU QIONG ET AL: "Chrysanolide A, an unprecedented sesquiterpenoid trimer from the flowers of Chrysanthemum indicum L", vol. 3, no. 26, 1 January 2013 (2013-01-01), GB, pages 10168 - 10172, XP093243269, ISSN: 2046-2069, Retrieved from the Internet <URL:https://doi.org/10.1039/c3ra23172k> DOI: 10.1039/c3ra23172k
- LI TIAN-ZE, YANG XIAO-TONG, WANG JIN-PING, GENG CHANG-AN, MA YUN-BAO, SU LI-HUA, ZHANG XUE-MEI, CHEN JI-JUN: "Biomimetic Synthesis of Lavandiolides H, I, and K and Artematrolide F via Diels–Alder Reaction", ORGANIC LETTERS, AMERICAN CHEMICAL SOCIETY, US, vol. 23, no. 21, 5 November 2021 (2021-11-05), US , pages 8380 - 8384, XP093048811, ISSN: 1523-7060, DOI: 10.1021/acs.orglett.1c03120
- ZHAO WEN-YU, YAN JUAN-JUAN, LIU TIAN-TIAN, GAO JIAN, HUANG HUI-LIAN, SUN CHENG-PENG, HUO XIAO-KUI, DENG SA, ZHANG BAO-JING, MA XIA: "Natural sesquiterpenoid oligomers: A chemical perspective", EUROPEAN JOURNAL OF MEDICINAL CHEMISTRY, ELSEVIER, AMSTERDAM, NL, vol. 203, 1 October 2020 (2020-10-01), AMSTERDAM, NL , pages 112622 - 22, XP093048812, ISSN: 0223-5234, DOI: 10.1016/j.ejmech.2020.112622
- HAI SHANG; JUNHUA LIU; RUIYANG BAO; YU CAO; KUN ZHAO; CHENGQIAN XIAO; BING ZHOU; LIHONG HU; YEFENG TANG: "Biomimetic Synthesis: Discovery of Xanthanolide Dimers", ANGEWANDTE CHEMIE INTERNATIONAL EDITION, VERLAG CHEMIE, HOBOKEN, USA, vol. 53, no. 52, 27 November 2014 (2014-11-27), Hoboken, USA, pages 14494 - 14498, XP072072002, ISSN: 1433-7851, DOI: 10.1002/anie.201406461
- KALIDINDI SRINIVAS, JEONG WON BOO, SCHALL ANDREAS, BANDICHHOR RAKESHWAR, NOSSE BERND, REISER OLIVER: "Enantioselective Synthesis of Arglabin", ANGEWANDTE CHEMIE INTERNATIONAL EDITION, VERLAG CHEMIE, HOBOKEN, USA, vol. 46, no. 33, 20 August 2007 (2007-08-20), Hoboken, USA, pages 6361 - 6363, XP093048813, ISSN: 1433-7851, DOI: 10.1002/anie.200701584
- SU LIHUA, ZHANG XINTIAN, MA YUNBAO, GENG CHANGAN, HUANG XIAOYAN, HU JING, LI TIANZE, TANG SHUANG, SHEN CHENG, GAO ZHEN, ZHANG XUEM: "New guaiane-type sesquiterpenoid dimers from Artemisia atrovirens and their antihepatoma activity", ACTA PHARMACEUTICA SINICA B, vol. 11, no. 6, 1 June 2021 (2021-06-01), pages 1648 - 1666, XP093048814, ISSN: 2211-3835, DOI: 10.1016/j.apsb.2020.12.006

## Description

### FIELD OF THE INVENTION

The present invention is in the field of pharmaceutical technol., and specifically relates to new guaianolide oligomers, a preparation method thereof, a pharmaceutical composition thereof and application thereof in the preparation of drugs against liver cancer.

### BACKGROUND OF THE INVENTION

Liver cancer, including primary liver cancer and secondary liver cancer, is one of the most common malignant tumors, hepatocellular carcinoma (HCC) accounts for more than 90% of liver cancer cases. Globally, HCC is the sixth most common malignant tumor. At present, there are more than 800,000 new cases of liver cancer in the world every year, and more than 460,000 cases in China every year. Liver cancer is characterized by insidious onset, long incubation period, strong invasiveness, high metastasis and other characteristics, and its degree of malignant is high. For patients with unresectable or advanced disease, the five-year survival rate is only 13%, and more than 422,000 people die from liver cancer every year in China. Clinically, four synthetic tyrosine kinase inhibitors (sorafenib, regorafenib, lenvatinib and cabozantinib) and three monoclonal antibody drugs (nivolumab, pembrolizumab and ramucirumab) have been approved for the treatment of HCC. These drugs play a significant therapeutic role in the clinic, but the disadvantages of these drugs such as low objective response rates (<20%), drug resistance, intolerance in some liver cancer patients and obvious side effects hamper their therapeutic effects.

Guaianolide oligomers are a class of natural products with complex structure and at least 30 carbon atoms that are formed by two or more guaianolide units. A large number of functional proteins exist as hetero- or homodimers or need to be activated by dimerization before mediating certain signaling pathways, and simultaneous targeting both monomeric moieties of dimeric proteins is an important strategy in drug development. Since sesquiterpenoid oligomers have the potential to target both monomers of dimer proteins, synthesis of sesquiterpenoid oligomers from anticancer active monomers is highly important. CN112225746 A discloses sesquiterpene dimers which can be used as anti-liver cancer drugs.

The present invention provides new guaianolide oligomers, and their preparation method and application. The synthetic method uses readily available material and is easy to operate and suitable for industrial production. This method has good substrate tolerance and can be used for the synthesis of guaianolide dimers, trimers or tetramers, providing a new synthetic method for the industrial preparation of guaianolide oligomers. So far, there are no reports about guaianolide oligomers 1-4, no reports about their synthetic methods, no reports about their use as active ingredients, and no reports about their use as anti-liver cancer drugs.

### SUMMARY OF THE INVENTION

The scope of the invention is defined by the claims. Any references in the description to methods of treatment refer to the compounds, pharmaceutical compositions and medicaments of the present invention for use in a method for treatment of the human (or animal) body by therapy (or for diagnosis).

The present invention provides new guaianolide oligomers **1-4** as represented by Formula I,

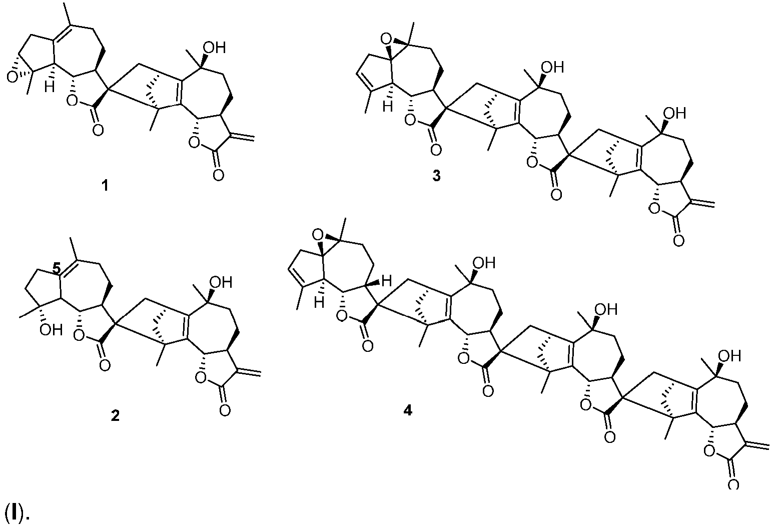

The invention also provides a preparation method for guaianolide oligomers, which comprises the preparation of guaianolide diene *via* an epoxy isomerization from arglabin-DMA, and subsequent Diels-Alder reaction between diene and sesquiterpenoids or oligomeric sesquiterpenoids containing α, β-unsaturated γ-lactone fragments/ deprotection of NMe₂ to obtain guaianolide oligomers. 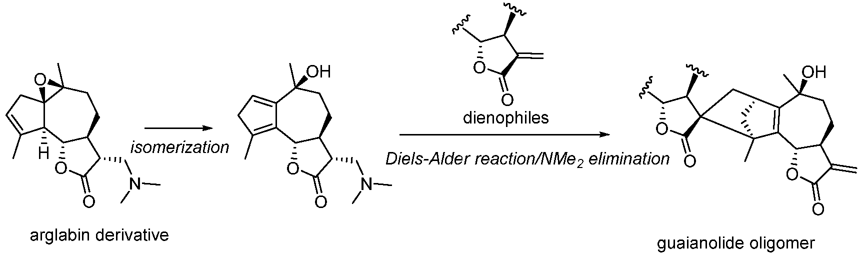 Wherein dienophiles include guaianolides such as ludartin, micheliolide, guaianolide dimers and trimers containing α, β-unsaturated γ-lactone fragments.

The preparation of guaianolide oligomers mainly includes the following two key reaction steps:
The first step: Diene was prepared from arglabin-DMA using an appropriate isomerizing reagent in an appropriate reaction solvent. Preferably, the isomerizing reagent used was diethylaluminum 2,2,6,6-tetramethylpiperidide (DATMP), Et₂AlN(c-Hex)₂, Et₂AlNPr*ⁱ*₂, Me₂Al(TMP) using toluene or benzene or diethyl ether or tetrahydrofuran as solvent; or Cp₂TiCl using toluene or benzene or diethyl ether or tetrahydrofuran as the solvent.
The second step: Diels-Alder reaction between diene and dienophiles containing α, β-unsaturated γ-lactone fragments under appropriate conditions and subsequent NMe₂ elimination to give guaianolide oligomers. The appropriate conditions for the Diels-Alder reaction include the diene reacted with the dienophiles under heating conditions using benzene, toluene, xylene and tetrahydrofuran as solvents, or that diene reacted with the dienophiles in the presence of 2,6-di-*tert-butyl-4-* methylphenol (BHT) under heating conditions using benzene, toluene, xylene and tetrahydrofuran as solvents, or that diene reacted with the dienophiles under neat condition at room temperature; or that dienes reacted with the dienophiles under neat condition at high temperature;

The invention also provides a pharmaceutical composition containing at least one of compounds 1-4 and pharmaceutically acceptable carriers and /or excipients. As used herein, the term "pharmaceutically acceptable carriers and /or excipients" refers to solid, semisolid, or liquid diluent, filler, or auxiliary formulation of any type. The invention has no special limitations on pharmaceutically acceptable carriers or excipients, carrier and/or excipient, which are well known in the field, and are nontoxic and inert to humans and animals.

The invention has no special limitation on the preparation method of the pharmaceutical composition, and can directly mix at least one of the compounds **1-4** with a pharmaceutically acceptable carrier or excipient. The invention has no special limitation on the mixing process, and the pharmaceutical composition can be obtained by selecting a known process in the field.

The invention provides an application of the pharmaceutical composition that mentioned above in the preparation of anti-liver cancer drugs. The invention has no special limitations on the method of application, and well known methods in the field can be selected.

In the invention, when the drug composition is used to prepare anti-liver cancer drug, the content of the composition in the drug is preferably 0.1 ∼99%; in the pharmaceutical composition, the content of at least one of the compounds **1-4** in the pharmaceutical composition is preferably 0.5 to 90%. The pharmaceutical composition of the invention is preferably used in the form of dosage per unit body weight. In the present invention, the prepared drug can preferably be administered by injection (intravenous injection, intramuscular injection) or oral administration.

### DESCRIPTION OF DRAWING

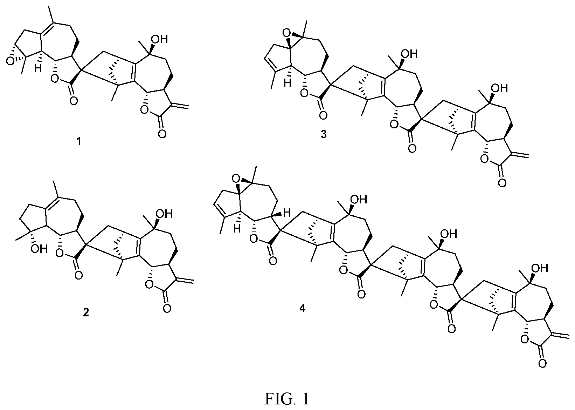

FIG. 1 The structural of guaianolide oligomers **1-4** of the invention.

### DETAILED DESCRIPITON OF THE INVENTION

The following examples describe the details of compounds and methods for understanding and supplementing the invention, but these examples are not intended and should not be constructed to limit in any way the invention set forth in the claims that follow thereafter.

### EXAMPLE 1

The synthesis of diene from arglabin-DMA

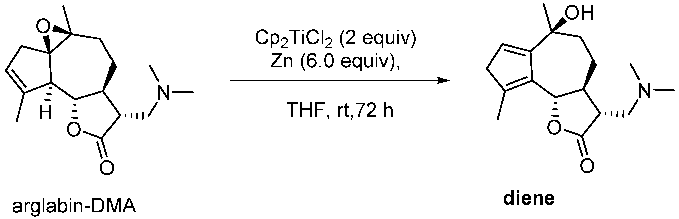

A mixture of Cp₂TiCl₂ (99.6 mg, 0.4 mmol, 2.0 equiv) and Zn (78 mg, 1.2 mmol, 6.0 equiv) in strictly deoxygenated THF (2 mL) was stirred at room temperature under an Ar atmosphere until the red solution turned green (after about 15 min). Arglabin-DMA (58.2 mg, 0.2 mmol, 1.0 equiv) was then added and the mixture was stirred for 72 h. The reaction was then quenched with a saturated solution of NaHCO₃(5 mL) and extracted with EtOAc (5 mL × 3). The organic layer was washed with brine, dried over anhydrous Na₂SO₄ and concentrated *in vacuo.* The crude product was purified by column chromatography on silica gel (eluted with Et₃N-EtOAc-petroleum ether, 1:20:80) to afford 45 mg of diene.

### Diene

Molecular formula: C₁₇H₂₅NO₃
Molecular weight: 291.39
Properties: Light yellow powder
[*α*]_{D}²³ -6.35 (c 0.145, methanol);
IR *v*ₘₐₓ 3434, 2934, 1772, 1458, 1369, 964 cm⁻¹;
HRMS (ESI, m/z): [M+H]⁺ calcd for [C₁₇H₂ₑNO_{3]}⁺ 293.1907, found 293.1889.

¹H NMR (500 MHz, CDCl₃) *δ* 5.98-5.97 (m, 1H, H-2), 5.35-5.33 (m, 1H, H-6), 2.80-2.79 (m, 2H, H-3), 2.70-2.66 (m, 1H, H-13a), 2.50-2.47 (m, 1H, H-13b), 2.40-2.35 (m, 1H, H-11), 2.24-2.22 (m, 1H, H-7), 2.16 (s, 6H, *N*-Me₂), 2.10 (d, *J* = 1.5 Hz, 3H, H-15), 2.03-1.88 (m, 4H, H-8a, H-8b, H-9a, H-9b), 1.51 (s, 1H, H-14); ¹³C NMR (125 MHz, CDCl₃) *δ* 177.9 (C-12), 151.1 (C-4), 136.9 (C-1), 135.1 (C-5), 124.1 (C-2), 82.9 (C-6), 70.5 (C-10), 58.5 (C-13), 49.7 (C-7), 45.8 (N-Me₂), 45.5 (C-3), 44.8 (C-11), 40.1 (C-9), 30.8 (C-14), 26.2 (C-8), 14.9 (C-15).

### EXAMPLE 2

### The synthesis of compound 1

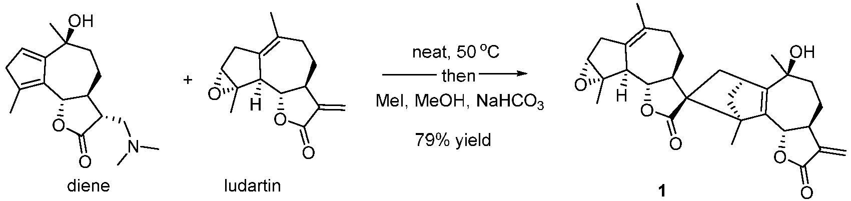

To the solution of ludartin (59 mg, 0.24mmol, 1.0 equiv) in CH₂Cl₂ (2 mL) was added compound diene (58 mg, 0.2 mmol, 1.0 equiv) at room temperature. After removal of the solvent under vacuum, the residue was heated to 50 °C for 60 h. After completion, the reaction mixture was dissolved in MeOH (5 mL) and treated with excess of Mel (62 µL, 1 mmol, 5 equiv) and stirred at room temperature. After 12 h, MeOH was removed under reduced pressure and the remained solid was taken in a separating funnel containing a mixture of 10% aqueous NaHCO₃ (5 mL) and EtOAc (5 mL). The mixture was then shaken until all the solid had dissolved and the organic phase was separated, the aqueous phase was extracted once again with EtOAc (2 × 5 mL). The combined organic phases were washed with brine, dried over Na₂SO₄ and evaporated under reduced pressure to give crude material. The crude product was then purified by column chromatography on silica gel (EtOAc-petroleum ether, 25:75) to afford compound 1 (78 mg, 79% yield).

### Compound 1

Molecular formula: C₃₀H₃₆O₆
Molecular weight: 492.61
Properties: white solid
Melting point:147.9-149.3 ºC
[*α*]_{D}²³ 33.70 (c 0.127, methanol)
IR *v*ₘₐₓ 3468, 2948, 1764, 1446, 1148, 1010, 543.8 cm⁻¹;
HRMS (ESI, m/z): [M+HCOO]⁻ calcd for [C₃₁H₃₇O₈]⁻ 537.2494, found 537.2462.

¹H NMR (500 MHz, CDCl₃) *δ* 6.15 (d, *J* = 3.5 Hz, 1H, H-11a), 5.54 (d, *J*=10.0 Hz, 1H, H-6), 6.15 (d, *J* = 3.5 Hz, 1H, H-11b), 3.59 (dd, *J =* 10.0, 10.0 Hz, 1H, H-6), 3.36 (brs, 1H, H-3), 3.04-3.01 (m, 2H, H-5', H-2), 2.69 (brd, *J =* 17.5 Hz, H-2'a ), 2.46-2.39 (m, 2H, H-2b, H-7), 2.24 (t, *J* = 13.5 Hz, 1H, H-9b), 2.15-2.10 (m, 2H, H-8a, H-13a'), 2.03-1.97 (m, 2H, H-9a, H-9b), 1.84-1.75 (m, 3H, H-9b, H-8b, H-8'a), 1.67 (s, 3H, H-14), 1.59 (s, 3H, H-15'), 1.52-1.44 (m, 2H, H-13', H-3a), 1.48 (s, 3H, H-15), 1.40 (s, 3H, H-14), 1.36-1.31 (m, 2H, H-8b, H-3b); ¹³C NMR (125 MHz, CDCl₃) *δ* 180.3 (C-12), 170.2 (C-12), 150.8 (C-1), 145.3 (C-5), 140.1 (C-11), 135.3 (C-10), 133.4 (C-1'), 119.0 (C-13), 83.8 (C-6), 77.5 (C-6), 72.7 (C-10), 67.3 (C-4), 63.8 (C-3'), 60.2 (C-4), 56.2 (C-11'), 55.5 (C-7), 53.5 (C-3), 51.8 (C-5), 46.8 (C-7), 42.9 (C-2), 38.6 (C-9), 35.1 (C-13), 33.8 (C-9), 33.2 (C-2), 27.6 (C-14), 25.9 (C-8), 23.6 (C-8), 22.1 (C-15), 19.3 (C-14), 17.0 (C-15);

### EXAMPLE 3

### The synthesis of compound 2

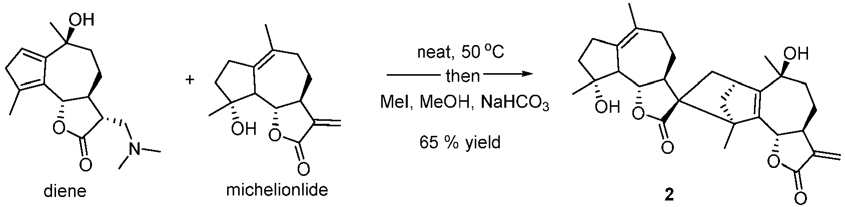

To the solution of michelionlide (60 mg, 0.24mmol) in CH₂Cl₂ (2 mL) was added diene (58 mg, 0.2 mmol, 1.0 equiv) at room temperature. After removal of the solvent under vacuum, the residue was heated to 50 °C for 60 h. After completion, the reaction mixture was dissolved in MeOH (5 mL) and treated with excess of Mel (62 µL, 1 mmol, 5 equiv) and stirred at room temperature. After 12 h, MeOH was removed under reduced pressure and the remained solid was taken in a separating funnel containing a mixture of 10 % aqueous NaHCO₃ (5 mL) and EtOAc (5 mL). The mixture was then shaken until all the solid had dissolved and the organic phase was separated, the aqueous phase was extracted once again with EtOAc (2 × 5 mL). The combined organic phases were washed with brine, dried over Na₂SO₄ and evaporated under reduced pressure to give crude material. The crude product was then purified by column chromatography on silica gel (EtOAc-petroleum ether, 30:70) to afford compound 2 (64 mg, 65% yield).

### Compound 2

Molecular formula:C₃₀H₃₀O₆
Molecular weight: 494.63
Properties: white solid
Melting point:93.8-94.6 ºC
[*α*]_{D} 4.13 (c 0.160, methanol)
IR *v*ₘₐₓ 3445, 2943, 1166, 1452, 1229, 1003, 675 cm⁻¹;
HRMS (ESI, m/z): [M+H]⁺ [C₃₀H₃₀O₆]⁺ calcd for 495.2741, found 495.2669.

¹H NMR (500 MHz, CDCl₃) *δ* 6.15 (d, *J =* 3.5 Hz, 1H, H-13a), 5.53 (d, *J =* 10.0 Hz, 1H, H-6), 5.43 (d, *J =* 3.5 Hz, 1H, H-13b), 5.30 (s, 1H, -OH), 3.75 (dd, *J =* 10.5, 10.5 Hz, 1H, H-6), 3.06-3.05 (m, 1H, H-2), 2.83-2.82 (m, 1H, H-7), 2.74 (s, 1H, -OH), 2.66-2.64 (m, 1H, H-5'), 2.40-2.35 (m, 2H, H-2'a, H-7), 2.22-2.11 (m, 7H, H-2b, H-9'a, H-9b, H-13'a, H-8'a, H-8a, H-9a), 1.86-1.73 (m, 5H, H-3'a, H-3b, H-8'B, H-8b, H-9b), 1.57 (s, 3H, H-14), 1.56-1.55 (s, 1H, H-3a), 1.52-1.51 (H-13b), 1.47 (s, 3H, H-15), 1.41 (s, 3H, H-14), 1.39-1.35 (m, 1H, H-3b) 1.29 (s, 3H, H-15); ¹³C NMR (125 MHz, CDCl₃) *δ* 180.3 (C-12), 170.2 (C-12), 151.0 (C-1), 145.2 (C-2), 140.2 (C-11), 132. 8 (C-1'), 131.7 (C-10), 119.0 (C-13), 83.7 (C-6), 81.2 (C-6), 80.8 (C-4), 72.7 (C-10), 60.6 (C-4), 58.3 (C-5), 56.2 (C-11), 53.9 (C-3), 51.7 (C-7), 47.0 (C-7), 42.9 (C-2), 38.8 (C-9), 38.5 (C-3), 35.3 (C-2), 34.9 (C-13), 29.9 (C-9), 27.4 (C-14), 25.3 (C-8'), 23.6 (C-8'), 23.5 (C-14'), 23.3 (C-15'), 17.5 (C-15).

### EXAMPLE 4

### The synthesis of compound 3

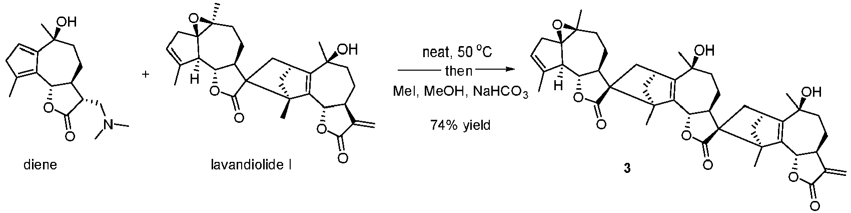

To the solution of lavandiolide l (118 mg, 0.24mmol) in CH₂Cl₂ (2 mL) was added diene (58 mg, 0.2 mmol, 1.0 equiv) at room temperature. After removal of the solvent under vacuum, the residue was heated to 50 °C for 60 h. After completion, the reaction mixture was dissolved in MeOH (5 mL) and treated with excess of Mel (62 µL, 1 mmol, 5 equiv) and stirred at room temperature. After 12 h, MeOH was removed under reduced pressure and the remained solid was taken in a separating funnel containing a mixture of 10 % aqueous NaHCO₃ (5 mL) and EtOAc (5 mL). The mixture was then shaken until all the solid had dissolved and the organic phase was separated, the aqueous phase was extracted once again with EtOAc (2 × 5 mL). The combined organic phases were washed with brine, dried over Na₂SO₄ and evaporated under reduced pressure to give crude material. The crude product was then purified by column chromatography on silica gel (EtOAc-petroleum ether, 40:60) to afford compound 3 (109 mg, 74% yield).

### Compound 3

Molecular formula:C₄₅H₅₄O₉
Molecular weight: 838.92
Properties: white solid
Melting point:137.9-139.2 ºC
[*α*]_{D}²⁴ 66.93 (c 0.150, methanol)
IR *v*ₘₐₓ 3468, 2939, 1165, 1452,1231, 969, 811 cm⁻¹;
HRMS (ESI, m/z): [M+HCOO]⁻ calcd for [C₄₆H₅₅O₁₁]⁻ 783.3750, found 783.3697.

¹H NMR (500 MHz, CDCl₃) *δ* 6.13 (d, *J* = 3.5 Hz, 1H, H-13a), 5.54-5.53 (m, 1H, H-3'), 5.51 (d, *J =* 10.0 Hz, 1H, H-6), 5.42 (d, *J =* 3.5 Hz, 1H, H-13b), 5.38 (d, *J* = 10.0 Hz, 1H, H-6'), 3.97 (dd, *J =* 10.0, 10.0 Hz, 1H, H-6'), 3.03-3.00 (m, 2H, H-2, H-2'), 2.75-2.71 (m, 1H, H-2"a), 2.59-2.54 (m, 1H, H-7'), 2.37 (brs, 1H, -OH), 2.30 (dd, *J =* 12.5, 3.5 Hz, 1H, H-13a), 2.18 (dd, *J* = 12.5, 3.5 Hz, 1H, H-13"a), 2.14-2.07 (m, 3H, H-2'b, H-8a, H-9"a), 2.01-1.95 (m, 3H, H-9'b, H-9'a, H-9a), 1.92-1.87 (m, 5H, H-7", H-8'a, H-15'), 1.84-1.64 (m, 4H, H-8b, H-9b, H-8b, H-9b), 1.60-1.55 (m, 2H, H-8"a, H-8'b), 1.51-1.40 (m, 7H, H-13'b, H-3b, H-13b, H-3b, H-15'), 1.39-1.26 (m, 14H, H-14", H-14', H-14, H-15, H-3b, H-3b); ¹³C NMR (125 MHz, CDCl₃) *δ* 181.3 (C-12'), 181.2 (C-12), 170.3 (C-12), 151.4 (C-1'), 151.1 (C-1), 144.6 (C-5), 144.4 (C-5), 140.8 (C-4'), 140.1 (C-11), 125.2 (C-3"), 119.0 (C-13), 84.1 (C-6), 81.5 (C-6'), 80.1 (C-6'), 72.4 (C-1‴), 72.3 (C-10), 72.1 (C-10), 62.1 (C-10'), 60.5 (C-4), 60.4 (C-4), 56.2 (C-11'), 55.6 (C-11"), 53.4 (C-3), 53.3 (C-3), 52.6 (C-5'), 52.4 (C-7'), 47.0 (C-7), 46.8 (C-7), 43.0 (C-2), 42.5 (C-2), 39.5 (C-9), 39.2 (C-2'), 38.5 (C-9), 35.1 (C-13), 34.7 (C-13'), 32.8 (C-9'), 27.8 (C-14), 27.2 (C-14), 23.9 (C-8), 22.6 (C-14"), 22.1 (C-8'), 20.9 (C-8'), 18.6 (C-15"), 17.6 (C-15), 17.4 (C-15).

### EXAMPLE 5

### The synthesis of compound 4

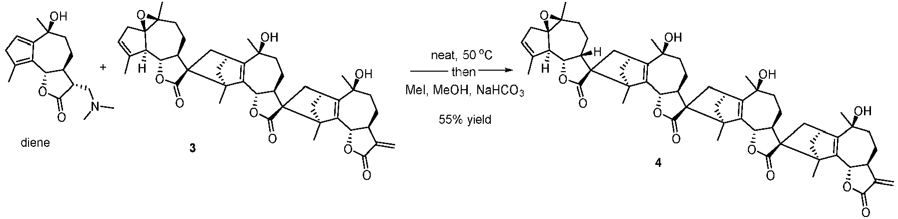

To the solution of compound 3 (88.6 mg, 0.12mmol) in CH₂Cl₂ (2 mL) was added diene (29 mg, 0.10 mmol, 1.0 equiv) at room temperature. After removal of the solvent under vacuum, the residue was heated to 50 °C for 60 h. After completion, the reaction mixture was dissolved in MeOH (5 mL) and treated with excess of Mel (31 µL, 0.5 mmol, 5 equiv) and stirred at room temperature. After 12 h, MeOH was removed under reduced pressure and the remained solid was taken in a separating funnel containing a mixture of 10 % aqueous NaHCO₃ (5 mL) and EtOAc (5 mL). The mixture was then shaken until all the solid had dissolved and the organic phase was separated, the aqueous phase was extracted once again with EtOAc (2 × 5 mL). The combined organic phases were washed with brine, dried over Na₂SO₄ and evaporated under reduced pressure to give crude material. The crude product was then purified by column chromatography on silica gel (EtOAc-petroleum ether, 50:50) to afford compound 4 (54 mg, 55% yield).

### Compound 4

Molecular formula:C₆₀H₇₂O₁₂
Molecular weight: 985.22
Properties: white solid
Melting point:157.7-158.4 °C
[*α*]_{D}²⁴ 91.23 (c 0.171,methanol)
IR *v*ₘₐₓ 3468, 2965, 1760, 1454, 1164, 1087, 739 cm⁻¹
HRMS (ESI, m/z): [M+H-H₂O]⁺ calcd for [C₆₀H₇₁O₁₁]⁻ 967.4996, found 967.4965

¹H NMR (500 MHz, CDCl₃) *δ* 6.14 (d, *J* = 3.5 Hz, 1H, H-13a), 5.55-5.54 (m, 1H, H-3"), 5.53 (d, *J =* 11.0 Hz, 1H, H-6), 5.43 (d, *J =* 3.5 Hz, 1H, H-13b), 5.37 (d, *J =* 10.5 Hz, 2H, H-6', H-6'), 3.97 (dd, *J =* 10.0, 10.0 Hz, 1H, H-6"), 3.03-3.00 (m, 3H, H-2, H-2', H-2'), 2.80-2.72 (m, 3-H, H-7, H-2‴a, H-5"), 2.60-2.52 (m, 2H, H-7', H-7'), 2.40 (brs, 1H, -OH), 2.32-2.26 (m, 3H, H-13'a, H-13"a, H-13‴a), 2.18-2.05 (m, 3H, H-2"b, H-9"b, H-8a), 2.02-1.96 (m, 4H, H-9"b, H-9"a, H-9'a, H-9a), 1.93-1.82 (m, 7H, H-7', H-15"', H-8"a, H-8'a, H-8b), 1.78-1.63 (m, 5H, H-9'b, H-8'b, H-9'b, H-8'b, H-9b, H-9b), 1.60-1.40 (m, 11H, H-8‴a, H-8‴b, H-13‴b, H-3"a, H-13"'b, H-3'a, H-13b, H-3a, H-15'), 1.39-1.27 (m, 21H, H-14‴, H-14", H-14', H-14, H-15, H-15', H-3b, H-3b, H-3'b); ¹³C NMR (125 MHz, CDCl₃) *δ* 181.4 (C-12"), 181.2 (C-12"), 181.2 (C-12), 170.3 (C-12), 151.9 (C-1 '), 151.4 (C-1'), 150.8 (C-1), 144.9 (C-5), 144.4 (C-5), 143.8 (C-5'), 140.8 (C-4"), 140.1 (C-11), 125.3 (C-3"), 119.0 (C-13), 84.1 (C-6), 81.6 (C-6'), 81.6 (C-6), 80.1 (C-6"), 72.4 (C-10'), 72.3 (C-10), 72.2 (C-10), 72.2 (C-1 ‴), 62.1 (C-10"), 60.6 (C-4'), 60.6 (C-4'), 60.4 (C-4), 56.2 (C-11 ‴), 55.9 (C-11 '), 55.6 (C-11'), 53.4 (C-3), 53.3 (C-3'), 52.9 (C-3"), 52.6 (C-7"), 52.5 (C-5"), 47.3 (C-7'), 47.0 (C-7), 46.9 (C-7), 43.1 (C-2), 42.8 (C-2), 42.5 (C-2'), 39.5 (C-9'), 39.4 (C-9), 39.2 (C-2‴), 38.6 (C-9), 35.1 (C-13"), 34.9 (C-13'), 34.7 (C-13), 32.9 (C-9"), 28.0 (C-14'), 24.0 (C-8), 22.6 (C-14‴), 22.2 (C-8'), 21.6 (C-8'), 20.9 (C-8"), 18.6 (C-15‴), 17.7 (C-15'), 17.6 (C-15), 17.4 (C-15).

### EXAMPLE 6:

In a previous study, sesquiterpenoid dimers isolated from *Artemisia atrovirens* showed inhibitory activity on three hepatoma cell lines (HepG2, Huh7, SMMC-7721), and the inhibitory effect of guaianolide oligomers 1-4 on hepatoma cell lines was further evaluated.

### 1. Materials and methods

### 1.1 Materials

HepG2, Huh7 and SK-HEP-1 cells were purchased from Shanghai Jining Biotechnology Co., LTD (Shanghai, China); Dulbecco's Modified Eagle Medium (DMEM) was purchased from Thermo Fisher Scientific (Suzhou, China); fetal bovine serum (FBS) was purchased from Life Technologies (NY, USA); RPMI-1640 was purchased from ThermoFisher Biochemical Products (Beijing, China)_{∘}

### 1.2 Instruments

Paradigm Molecular Devices (Bio-RAD 680, USA); Analytical balance (AG135, Metler Toledo, China); Incubator (DHP-9082, Shanghai)_{∘}

### 1.3 Experimental Process

(1). Hepatocellular carcinoma cells at several stages of growth were collected and washed twice with PBS, after which PBS was discarded;
(2). The cells were digested with 0.25% trypsin, and the trypsin was removed when the cell contour was observed to be deepened and rounded under the microscope;
(3) DMEM containing 10% FBS was used to terminate the digestion and suspend the cells, 10 µL of cell suspension was taken and counted by cell counter, and the cell concentration was adjusted to 1 × 10⁴/mL in the medium. 100 µL of cell suspension was seeded into 96-well plates. The cells were incubated in a 5% CO₂ incubator for 24 h to allow the cells to adhere to the wall.
(4). The culture medium was replaced with fresh medium containing different concentrations of compounds, and the cells were incubated for an additional 48 h;
(5). After removal of the medium, 100 µL of MTT reagent (1 mg/mL) was added to each well, and the plates were kept in an incubator for 4 h.
(6). Dimethyl sulfoxide (DMSO, Solarbio, Beijing, China) was added to dissolve the MTT formazan salt
(7). The plates were measured at 490 nm using a microplate reader (BIO-RAD, USA). The inhibitory ratio was calculated as [(A490 negative - A490 treated)/(A490 negative - A490 blank)] × 100%. The IC₅₀ values were calculated with GraphPad Prism 5 (GraphPad Software, California, USA). Repeat the experiment three times.

### 2. Results

The inhibitory activities of compounds 1-4 on HepG2, Huh7 and SK-Hep-1 cells are shown in Table 3, and all four compounds showed obvious activity against three HCC cell lines with IC₅₀ values ranging from 6.2 to 40.4 µM. Among them, trimeric compound 3 was the most active, with IC₅₀ values of 6.2, 6.8, 7.2 µM, which were superior to those of positive control drug sorafenib.

**Table 3. Antihepatoma activities of compounds 1-4**

| compounds | IC₅₀ (µM)^{a} | | |
|---|---|---|---|
| | HepG2 | Huh7 | SK-Hep-1 |
| 1 | 12.8 | 20.2 | 11.2 |
| 2 | 5.9 | 11.9 | 16.5 |
| 3 | 6.2 | 6.8 | 7.2 |
| 4 | 14.7 | 10.9 | 7.9 |
| sorafenib | 12.8 | 10.9 | 22.5 |

### 3. Conclutions

These results indicate that the new oligomeric guaianolides 1-4 showed inhibitory activities on three hepatocellular carcinoma cell strains (HepG2, Huh7 and SK-Hep-1) and can be used as drugs for treating hepatocellular carcinoma-related diseases.

### Example of formulation:

In the following examples of formulation, conventional reagents are selected, and formulations are prepared according to existing conventional methods, and the example only embodies that at least one of the compounds 1-4 described in the present invention can be prepared into different formulations without specific limitations on reagents and operations:
1. At least one of the compounds 1-4 was dissolved in DMSO and dispersed in sterile water for injection according to the conventional method, finely filtered, and sterilized and sealed to make an injection solution, and the concentration of injection solution was 0.5~5 mg/mL.
2. At least one of the compounds 1-4 was dissolved in DMSO, dispersed in sterile water for injection, stirred to dissolve, filtered in a sterile extraction funnel, then finely filtered, subpacked in ampoules, freeze-dried at low temperature, aseptically sealed to give a powder injection.
3. At least one of the prepared compounds 1-4 was mixed with excipient at a mass ratio of 9:1 to make powder.
4. At least one of the prepared compounds **1-4** was mixed with excipient at a mass ratio of 5:1 and then granulated and pressed to obtain a tablet.
5. At least one of the prepared compounds **1-4** is made into an oral liquid *via* a conventional method.
6. At least one of the prepared compounds **1-4** was mixed with excipient at a mass ratio of 5:1 to make a capsule.
7. At least one of the prepared compounds **1-4** was mixed with excipient at a mass ratio of 5:1 to make a granule formulation.

From the above embodiments, it is clear that the present work provides new guaianolide oligomers, and their preparation methods and applications. The new guaianolide oligomers showed obvious inhibitory activity on hepatoma carcinoma cells, and can be used in drug compositions with medicinal carriers and/or excipients, and can be used to prepare anti-liver cancer drugs.

The above is only a preferred embodiment of the present invention, and it should be noted that for technicians in the medicinal field, several improvements and refinements can be made without deviating from the principle of the invention, and these improvements and refinements should also be considered as the scope of protection of the invention.

## Claims

1. guaianolide oligomers **1-4** as represented by Formula I,

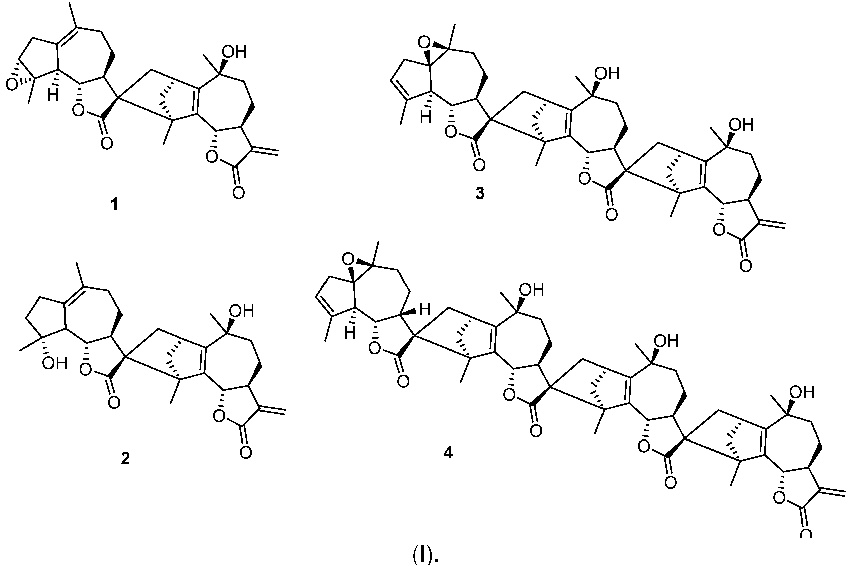

2. A guaianolide diene as represented by Formula II for the preparation of guaianolide oligomers,

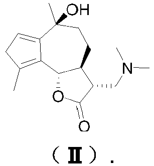

3. A method for preparing the guaianolide oligomers according to claim 1, which comprises a preparation of guaianolide diene via epoxy isomerization from arglabin-DMA, and a Diels-Alder reaction between the guaianolide diene and dienophiles/ deprotection of NMe₂ to obtain the guaianolide oligomers, wherein dienophiles include guaianolides such as ludartin, micheliolide, guaianolide dimers and trimers containing α, β-unsaturated γ-lactone fragments,

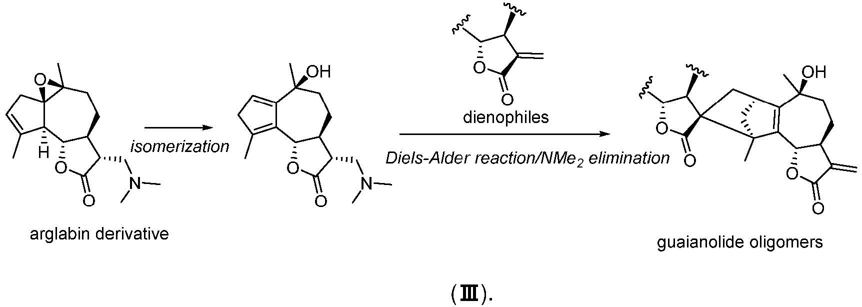

4. The method according to claim 3, wherein the method mainly includes the following two key reaction steps:
The first step: Diene was prepared from arglabin-DMA using an appropriate isomerizing reagent in an appropriate reaction solvent; Preferably, the isomerizing reagent used were diethylaluminum 2,2,6,6-tetramethylpiperidide (DATMP), Et₂AlN(c-Hex)₂, Et₂AlNPr*ⁱ*₂, Me₂Al(TMP) using toluene or benzene or diethyl ether or tetrahydrofuran as the solvent; or Cp₂TiCl using toluene or benzene or diethyl ether or tetrahydrofuran as the solvent;
The second step: The Diels-Alder reaction between diene and dienophiles containing α, β-unsaturated γ-lactone fragments under appropriate conditions and subsequent NMe₂ elimination to give guaianolide oligomers, the appropriate condition for the Diels-Alder reactions are that the diene reacted with the dienophiles under heating conditions using benzene, toluene, xylene and tetrahydrofuran as solvents, or that diene reacted with the dienophiles in the presence of 2,6-di-*tert-butyl-4-* methylphenol (BHT) under heating conditions using benzene, toluene, xylene and tetrahydrofuran as solvents, or that diene reacted with the dienophiles under neat condition at room temperature; or that the dienes reacted with the dienophiles under neat condition at high temperature.

5. Application of the guaianolide oligomers 1-4 according to claim 1 in the preparation of anti-liver cancer drugs.

6. A pharmaceutical composition containing at least one of the guaianolide oligomers 1-4 according to claim 1 and pharmaceutically acceptable carriers and /or excipients.

7. Application of the pharmaceutical composition according to claim 6 in the preparation of anti-liver cancer drugs.

## Patentansprüche

1. Guaian-Sesquiterpen-Polymere, deren Struktur durch die Formel (I) dargestellt wird,

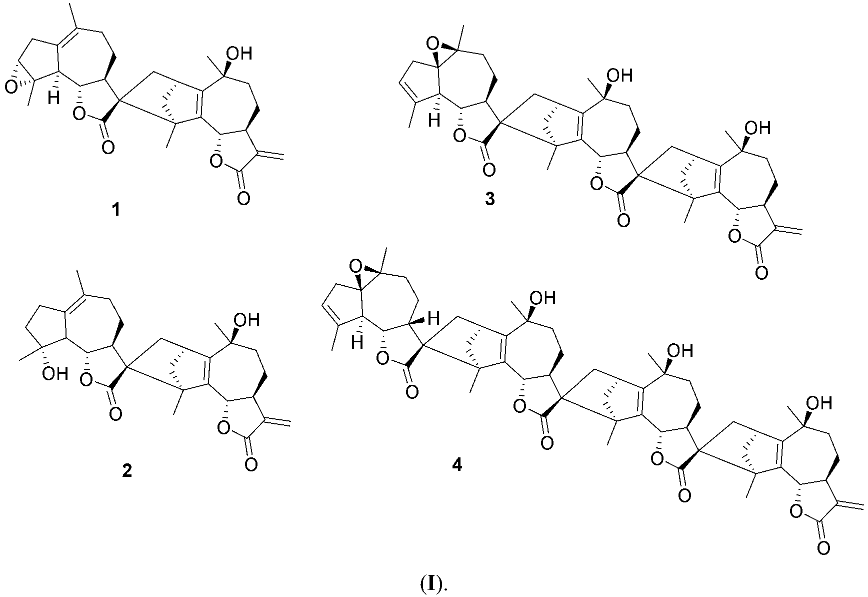

2. Guaian-Dien, das durch die Formel (II) dargestellt wird und zur Herstellung von Guaian-Sesquiterpen-Polymeren verwendet wird.

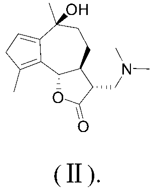

3. Verfahren zur Herstellung der Guaian-Sesquiterpen-Polymere nach Anspruch 1, umfassend die Epoxid-Isomerisierung von Dimethylamino-Arglabin (Arglabin-DMA) als Ausgangsmaterial zum Guaian-Dien, gefolgt von einer Diels-Alder-Reaktion/Entschützung des Dimethylamins des Diens mit einem Dienophil zur Herstellung des Guaian-Sesquiterpen-Polymers; wobei das Dienophil mindestens eines der folgenden umfasst: Guaian-Sesquiterpene wie Ludartin und Michelia-Lacton, Guaian-Sesquiterpen-Dimere oder -Trimere, die ein α,β-ungesättigtes γ-Lacton-Fragment enthalten,

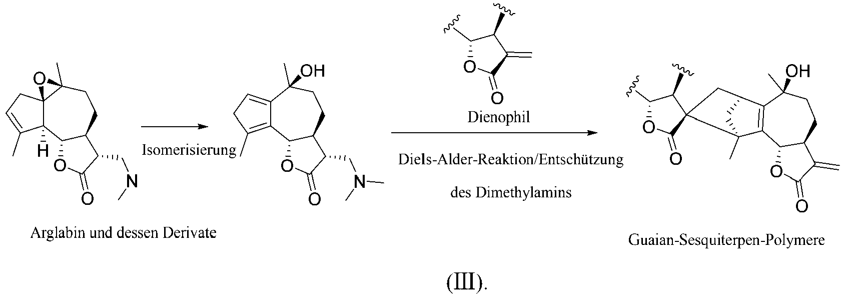

4. Verfahren zur Herstellung der Guaian-Sesquiterpen-Polymere nach Anspruch 3, **dadurch gekennzeichnet, dass** das Syntheseverfahren für Guaian-Sesquiterpen-Polymere hauptsächlich die folgenden zwei Schlüsselreaktionsschritte umfasst:
Reaktionsschritt eins: Dimethylamino-Arglabin wird in einem geeigneten Reaktionslösungsmittel mit einem geeigneten Isomerisierungsreagenz umgesetzt, um das Dien zu erhalten; wobei das Isomerisierungsreagenz 2,2,6,6-Tetramethylpiperidino-diethylaluminium oder Dicyclohexyl aminoaluminium oder Diisopropylamino diethylaluminium oder 2,2,6,6-Tetramethylpiperidino-dimethylaluminium ist, und das Reaktionslösungsmittel Toluol, Benzol, Diethylether oder Tetrahydrofuran ist; oder, das Isomerisierungsreagenz ist Titanocendichlorid, und das Reaktionslösungsmittel ist Toluol, Benzol, Diethylether oder Tetrahydrofuran.
Reaktionsschritt zwei: Das Dien wird unter geeigneten Bedingungen mit einem Dienophil, das ein α,β-ungesättigtes γ-Lacton-Fragment enthält, einer Diels-Alder-Reaktion/Entschützung des Dimethylamins unterzogen, um das Sesquiterpen-Polymer herzustellen, wobei die geeigneten Bedingungen für die Diels-Alder-Reaktion sind: Benzol, Toluol, Xylol oder Tetrahydrofuran als Lösungsmittel, und Reaktion des Diens mit dem Dienophil unter Erwärmung; oder
2,6-Di-tert-butyl-4-methylphenol als Antioxidans, Benzol, Toluol, Xylol oder Tetrahydrofuran als Lösungsmittel, und Reaktion des Diens mit dem Dienophil unter Erwärmung; oder, Reaktion des Diens mit dem Dienophil lösungsmittelfrei bei Raumtemperatur; oder, Reaktion des Diens mit dem Dienophil lösungsmittelfrei unter Erwärmung.

5. Verwendung der Guaian-Sesquiterpen-Polymere nach Anspruch 1 zur Herstellung eines Arzneimittels gegen Leberkrebs.

6. Pharmazeutische Zusammensetzung, umfassend mindestens eines der Guaian-Sesquiterpen-Polymere 1-4 nach Anspruch 1 und einen pharmazeutisch akzeptablen Träger oder ein pharmazeutisch akzeptables Exzipiens.

7. Verwendung der pharmazeutischen Zusammensetzung nach Anspruch 6 zur Herstellung eines Arzneimittels gegen Leberkrebs.

## Revendications

1. Polymère sesquiterpénique à base de guaiane, **caractérisé en ce qu'**il comprend une formule structurale représentée par la formule (I),

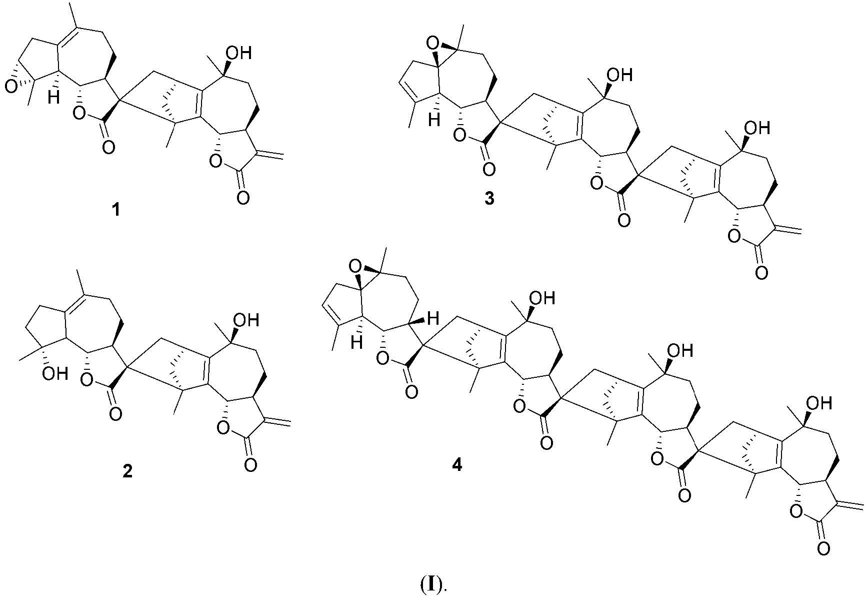

2. Diène de guaiane indiqué dans la formule (**II**), **caractérisé en ce qu'**il est applicable dans la préparation du polymère sesquiterpénique à base de guaiane,

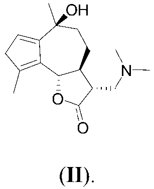

3. Procédé de préparation du polymère sesquiterpénique à base de guaiane selon la revendication 1, **caractérisé en ce qu'**il consistant à adopter le diméthylamino-arglabine (arglabine-DMA) comme le produit de départ pour obtenir un diène du type de guaïane par isomérisation d'époxyde, à obtenir ainsi le polymère sesquiterpénique à base de guaiane après la réaction Diels-Alder entre le diène et le diénophile ou après la préparation de dédiméthylamination ; le diénophile étant choisi parmi au moins l'un des composés suivants : un sesquiterpène du type de guaiane tel que le ludartin ou le micrantholid, un dimère ou un trimère sesquiterpénique du type de guaiane avec fragment α,β-insaturé-γ-lactone ;

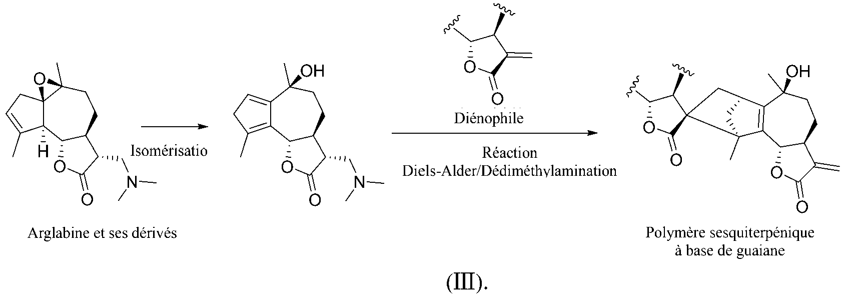

4. Procédé de préparation du polymère sesquiterpénique à base de guaiane selon la revendication 3, **caractérisé en ce que** la méthode de synthèse comprenant essentiellement les deux étapes de réaction importantes suivantes :
Étape de réaction 1 : L'arglabine-DMA étant mis à réagir avec un réactif d'isomérisation approprié dans un solvant de réaction adapté pour obtenir un diène ; ledit réactif d'isomérisation étant choisi parmi le 2,2,6,6-tétraméthylpipéridinyl-diéthylaluminium ou le dicyclohexylaminodiétylaluminium ou le diisopropylamino-diétylaluminium ou le 2,2,6,6-tétraméthylpipéridinyl-diméthylaluminium ; le solvant de réaction étant choisi parmi le toluène, le benzène, l'éther diéthylique ou le tétrahydrofurane ; ou bien, le réactif d'isomérisation étant le monochlorotitanocène ; le solvant de réaction étant choisi parmi le toluène, le benzène, l'éther diéthylique ou le tétrahydrofurane ;
Étape de réaction 2 : le polymère sesquiterpénique étant obtenu après la réaction Diels-Alder entre le diène et le diénophile avec fragment α,β-insaturé-γ-lactone ou après la préparation de dédiméthylamination dans les conditions appropriées ; les conditions appropriées pour la réaction de Diels-Alder impliquant l'utilisation de benzène, de toluène, de xylène ou de tétrahydrofurane à titre de solvants ; le diène et le diénophile réagissant dans des conditions de chauffage ; ou le 2,6-di-tert-butyl-4-méthylphénol étant utilisé comme agent antioxydant, en présence de benzène, de toluène, de xylène et de tétrahydrofurane à titre de solvants ; le diène et le diénophile réagissant dans des conditions de chauffage ; en variante, le diène et le diénophile réagissant à température ambiante dans en cas d'absence d'un solvant ; en variante, le diène et le diénophile réagissant dans des conditions de chauffage en cas d'absence d'un solvant.

5. Polymère sesquiterpénique à base de guaiane selon la revendication 1, **caractérisé en ce qu'**il est applicable dans la préparation de médicaments contre le cancer du foie.

6. Polymère sesquiterpénique à base de guaiane 1-4 selon la revendication 1, **caractérisé en ce qu'**il comprend au moins une composition pharmaceutique avec un support ou un excipient acceptable dans le domaine pharmaceutique.

7. Composition pharmaceutique selon la revendication 6, **caractérisée en ce qu'**elle est applicable dans la préparation de médicaments contre le cancer du foie.
